# EUROPEAN PATENT APPLICATION

(11) **EP 3 357 422 A1**
(43) Date of publication of application: **08.08.2018**
(21) Application number: 17154928.0
(22) Date of filing: 07.02.2017
(51) Int. Cl.: A61B 5/157, A61M 31/00, A61B 5/145, A61B 5/15, A61M 37/00, A61B 5/155

(54) **MICRONEEDLES AND INSERTABLE DEVICES WITH INTEGRATED ANTENNA ARRAY**

(71) Applicant: Koninklijke Philips N.V., 5656 AE Eindhoven (NL)
(72) Inventor: VISWESWARA, Ashoka Sathanur, 5656 AE Eindhoven (NL); ESPINA PEREZ, Javier, 5656 AE Eindhoven (NL); JOHNSON, Mark Thomas, 5656 AE Eindhoven (NL); MEFTAH, Mohammed, 5656 AE Eindhoven (NL)
(74) Representative: de Haan, Poul Erik

(57) **Abstract**

Various methods and apparatus for a wearable or insertable device (100, 200, 400, 600) with microneedles (106, 206, 306, 406, 606) that may be simultaneously used for radio frequency (RF) energy harvesting, communication, and transdermal fluid delivery and collection are disclosed. Such a device may comprise a substrate (102, 202, 302, 402, 602) that may be affixable/insertable into tissue (107, 607) of a patient, radio frequency circuitry (120, 220, 420, 620) disposed on the substrate, that may generate/process a signal with a frequency that may be modulated to carry information, and microneedles (106, 206, 306, 406, 606) extending from at least one surface of the substrate, where one or more microneedles define a micro-fluidic channel (130, 230, 430, 630) that fluidly couples the tissue with a conduit (132) of the substrate, and where one or more microneedles may be electrically coupled with the radio frequency circuitry and radiate/process electromagnetic waves based on the signal.

## Description

### FIELD OF THE INVENTION

The present disclosure is directed generally to wearable and insertable electronic devices and components thereof. More particularly, but not exclusively, the various apparatus, methods, and systems disclosed herein relate to microneedles that are simultaneously used for radio frequency (RF) energy harvesting, communication, and/or transdermal fluid delivery and/or collection.

### BACKGROUND OF THE INVENTION

In 2001, the Federal Communications Commission (FCC) allocated 7 GHz in the 57-64 GHz band ("the 60GHz band") for unlicensed use. This allocation, in combination with advances in wireless communication technologies, allows for the possibility of miniaturized radio frequency (RF) systems. The 60 GHz band is of particular interest because of the specific attenuation characteristic caused by atmospheric oxygen of 10 to 15dB/km, which makes the 60 GHz band unsuitable for long-range, for example longer than two kilometers, communications. Thus, the 60 GHz band can be dedicated entirely to short-range, for example less than one kilometer, communications.

Generally, high frequencies are associated with both advantages and disadvantages. High propagation attenuation at 60GHz classifies a set of short-range applications, but also indicates dense frequency reuse patterns. Higher frequencies lead to a miniaturization of sizes of RF components, including antennas. At millimeter wave (MMW) frequencies (between 30GHz to 300GHz), not only are the antennas very small, but they may also be quite directional, which may be desired. Typically, high antenna gains for 60 GHz systems come, in part, from the Friis Equation for Path Loss. This equation, also known as Friis' Law, holds that as the frequency of operation increases, the effective area of any particular antenna decreases proportionately to the frequency squared. With the continued miniaturization of antennas for 60GHz and other high frequency bands, on-chip silicon antenna arrays may be designed to provide excellent directionality. This miniaturization and possibility for phased array antennas with high gain may make 60GHz antennas extremely efficient as compared to traditional antennas in industrial, scientific, and medical radio frequency bands.

Reliable microneedles have broad applications to fluid sampling, fluid delivery, and precisely localized chemical-reaction stimulation. Where robust enough to penetrate biological tissue, microneedle applications may have many biomedical uses. Where microneedles are fabricated from an integrated circuit (IC) compatible process they have a distinct advantage owed to the feasibility of on-chip electronics which may form a part of a chemical design and-analysis system and/or may drive various electronics for pumps, valves, and the like. It has been proposed that microneedles of about 150µm in length could cross the stratum corneum permeability barrier to enhance transdermal drug delivery, and it has been further hypothesized that this needle insertion should be painless given that the needles are not long enough to reach the nerves.

### SUMMARY OF THE INVENTION

The present disclosure is directed to methods and apparatus for wearable or insertable devices with microneedles that are simultaneously used for radio frequency (RF) energy harvesting, communication, and transdermal fluid delivery and collection. Generally, in one aspect a wearable or insertable device is disclosed, where the device contains: a substrate that is affixable to or insertable into tissue of a patient; radio frequency circuitry disposed on the substrate, where the radio frequency circuitry is operable to generate (transmit) or process (receive) a signal with a frequency that is modulated to carry information; and, an array of microneedles extending from at least one surface of the substrate, where one or more microneedles of the array of microneedles defines a micro-fluidic channel that fluidly couples the tissue of the patient with a conduit of the substrate, and where one or more microneedles of the array of microneedles are electrically coupled with the radio frequency circuitry and radiate or process electromagnetic waves based on the signal.

In some embodiments, the substrate comprises a multi-layer silicon substrate that includes a first layer with the radio frequency circuitry and a second layer that supports the array of microneedles. In other embodiments, the wearable or insertable device further comprises one or more through silicon vias that fluidly couple the micro-fluidic channels of the array of microneedles through the first layer to the conduit. In still other embodiments, the conduit leads to an additional layer of the multi-layer silicon substrate that is a fluid analysis layer that analyzes fluid extracted through the micro-fluidic channels of the array of microneedles. In additional embodiments, the conduit leads to an additional layer of the multi-layer silicon substrate that is a fluid chamber containing fluid for injection into tissue via the micro-fluidic channels of the array of microneedles.

In some embodiments, the array of microneedles are formed as a phased antenna structure that combines signals from one or more of the plurality of microneedle arrays to achieve beamforming of at least some of the electromagnetic waves. In other embodiments, the plurality of microneedles are oriented perpendicularly to the substrate. In still other embodiments, the plurality of microneedles are oriented parallel to the substrate. In some embodiments, the plurality of microneedles are laterally oriented with respect to a patient's skin surface. In other embodiments, the array of microneedles are arranged in concentric rings following a profile of a corrugated horn antenna.

In some embodiments, each of the plurality of microneedles further comprise a metal coating. In some embodiments, the metal coating is applied to an interior surface of the micro-fluidic channel of each of the plurality of microneedles. In other embodiments, the metal coating is applied to an exterior surface of each of the plurality of microneedles. In some embodiment, at least some of the plurality of microneedles extend through the silicon substrate by way of metallic vias. In other embodiments, the antenna element is extended to a length of about 250 to about 1500 microns.

In some embodiments, the wearable or insertable device further comprises a fluid detection sensor that detects presence of fluid in the plurality of microneedles and activates the radio frequency circuitry when fluid is present. In other embodiments, the radio frequency circuitry processes the signal in response to a determination, based on a signal from the fluid detection sensor, that there is no fluid in the micro-fluidic channels of the array of microneedles.

Generally, in another aspect a method is described, where the method includes: obtaining an insertable device, where the device comprises a substrate that is affixable to or insertable into tissue of a patient, radio frequency circuitry disposed on the substrate, where the radio frequency circuitry is operable to generate or receive a signal a frequency that is modulated to carry information, and an array of microneedles extending from at least one surface of the substrate, where one or more microneedles of the array of microneedle defines a micro-fluidic channel that fluidly couples the tissue of the patient with a conduit of the substrate, and where one or more microneedles of the array of microneedles are electrically coupled with the radio frequency circuitry and radiate electromagnetic waves based on the signal; inserting the obtained device into the tissue of a patient; orienting the array of microneedles such that at least some of the array of microneedles are oriented with a tip of each microneedle pointing to a surface of the patient's skin; and implanting the device so as to maintain orientation of the tips of one or more microneedles pointing to the surface of the patient's skin.

In some embodiments, the device further comprises a fluid detection sensor that detects presence of fluid in one or more of the plurality of microneedles and signals the radio frequency circuitry when fluid is present. In other embodiments, the method further comprises generating, by the radio frequency circuitry, the signal in response to determining, based on a signal from the fluid detection sensor, that there is no fluid in the microfluidic channels of one or more microneedles of the array of the microneedles.

### BRIEF DESCRIPTION OF THE DRAWINGS

In the drawings, like reference characters generally, but not exclusively, refer to the same parts throughout the different views. Also, the drawings are not necessarily to scale, emphasis instead generally being placed upon illustrating the principles of the disclosure.
Figure 1 illustrates a cross-section of human skin with an embodiment of a wearable device.
Figure 2 illustrates a cross-section of human skin with an embodiment of an insertable device.
Figure 3 illustrates an embodiment of the device where an array of microneedles are arranged in concentric circles.
Figure 4 illustrates a cross-sectional view of an embodiment of a wearable or insertable device.
Figure 5 depicts an example method of implanting an insertable device into the tissue of a patient.
Figure 6 depicts an example of an insertable device configured with selected aspects of the present disclosure, in accordance with various embodiments.

### DETAILED DESCRIPTION OF EMBODIMENTS

Wearable/insertable devices are becoming more accepted in clinical settings, and home monitoring is becoming an increasingly important feature of healthcare systems. Wearable and/or insertable devices have traditionally been used to measure vital signs such as heart rate, respiration rate, temperature, oxygen saturation, and so forth. There is also an increasing body of research into using these devices to measure bioanalytes and for use in drug delivery. There is a need for wearable or insertable devices to be able to wirelessly communicate and exchange data with one or more remote computing devices. Such communication may allow the wearable/insertable device to communicate with another device of the patient (e.g. a smart phone or smart watch), devices belonging to caregivers, other monitoring devices, and the like. While the desire for wireless communication is recognized, conventional wireless systems typically require hardware that is often bulky, which may require the host device to be larger than would otherwise be necessary. The use of radio frequency circuitry capable of generating electrical current at frequencies used to carry information incorporated into the wearable/insertable device itself may allow for miniaturized wearable/insertable devices capable of wireless communication.

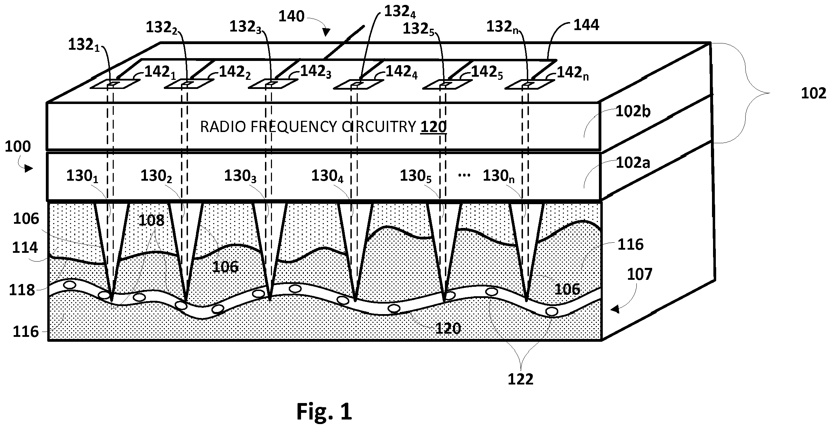

Referring to Figure 1, in one embodiment, an apparatus 100 for affixing to or inserting into the tissue 107 of a patient, is illustrated. In the illustrated embodiment, the wearable device is in the form a thin patch or tattoo-like structure with a silicon substrate 102, radio frequency circuitry 120 that is incorporated as a part of the silicon substrate, a plurality of microneedles 106, and a plurality of micro-fluidic channels 130₁, 130₂, 130₃, and so on. While examples described herein refer to the substrate as being "silicon," this is not meant to be limiting. Substrate 102 may be constructed with other materials in addition to or instead of silicon. In various embodiments, the wearable device 100 may be affixed to a patient by means of silicon substrate 102. For example, microneedles 106 disposed on the silicon substrate 102 (e.g., a bottom side in Figure 1) may be inserted (e.g., pierced) through tissue 107, which in some cases may be the surface of the patient's skin. Assuming that is the case, tissue 107 may include an epidermis 114 separated from a dermis 116 by an epidermal-dermal junction ("EDJ") 118. The tips 108 of microneedles 106 may reach one or more capillaries 120 (which may carry arterial or venous blood).

In some embodiments, such as the wearable device 100 illustrated in Figure 1, the silicon substrate 102 may include multiple layers. The substrate may include a layer 102b that the radio frequency circuitry 120 is incorporated into, such that the radio frequency circuitry 120 may generate an electrical current with a frequency modulated to carry information. Radio waves have varying frequencies, and these various frequencies are capable of transmitting data at differing qualities and distances. For example, high propagation attenuation at about 60GHz may be best suited for short-range applications, but may also indicate dense frequency reuse patterns. Furthermore, higher frequencies may lead to a miniaturization of sizes of radio frequency components, including antennas. Therefore, it may be desirable for wearable/insertable devices to communicate at higher radio frequencies, for example by making use of the 60GHz band. Such frequencies would allow the wearable/insertable device(s) to maintain a small size, while simultaneously allowing the device(s) to wirelessly communicate and exchange data with one or more remote computing devices. This exchange of data may be beneficial for sending information to and receiving information from the wearable/insertable device. Some examples of this sending/receiving information include, but are not limited to: sending measurements of biomarkers measured from a patient's fluid or tissue (e.g. blood counts, glucose levels, cholesterol levels, etc.) to a remote device for review by the patient or clinician; or, receiving information regarding drug dosages to inject into a patient's tissue (e.g. amount of insulin) from a remote device controlled by the patient, clinician, etc. It is to be understood that the foregoing examples are not limiting and are merely illustrative.

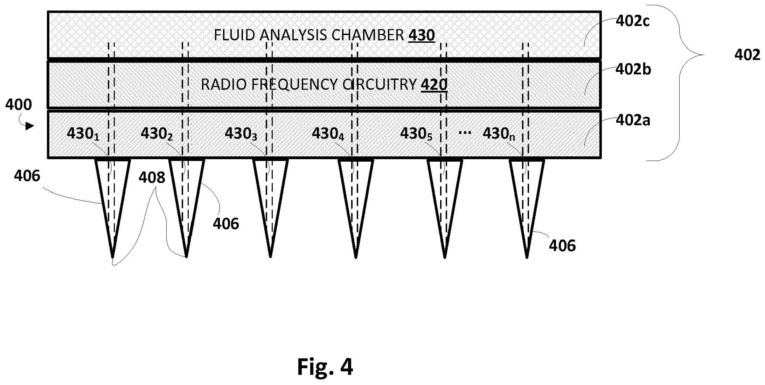

In some embodiments, the substrate 102 may further include another layer 102a that supports the microneedles 106. The microneedles 106 themselves may be fabricated on the silicon substrate, e.g., on layer 102a, and each may define a micro-fluidic channel 130 that fluidly couples the tissue 107 of the patient with a corresponding conduit 132 (e.g., a fluid interface, port, opening, etc.) of the silicon substrate 102. The conduits 132₁₋ₙ may provide a fluid interface to, for instance, an additional fluid analysis layer (see Figure 4) and/or fluid pathways (e.g., tubing, hoses, etc.) to other destinations, such as a remote fluid analysis system. Each micro-fluidic channel 130: may allow for the collection of fluid from the tissue of a patient, for example blood; may allow for the insertion of fluid into the tissue, for example a pharmaceutical; or, may be used to both collect and insert varying fluids into the tissue of a patient. Furthermore, the microneedles 106 may be electrically coupled with the radio frequency circuitry 120 and radiate electromagnetic waves based on electrical currents generated by the radio frequency circuitry. In other words, one or more of the microneedles 106 may also function as an antenna.

The orientation of each of the microneedles/antennas 106 relative to the substrate 102 and the surface of the patient's skin may vary depending on the type and use of the device. For example, certain arrangements of the microneedle/antenna may enhance wireless communication with remote devices. In some embodiments, such as illustrated in Figure 1, the microneedles 106 may be oriented perpendicular to the silicon substrate 102. Furthermore, the microneedles may also be oriented perpendicular to the tissue 107 of a patient, for example the surface of a patient's skin. This configuration may be typical where the device is wearable and/or penetrates the skin surface. In these embodiments, the microneedles/antenna may penetrate the skin of the patient from the outer dermal layers, while the silicon substrate 102 remains on the exterior surface of the skin.

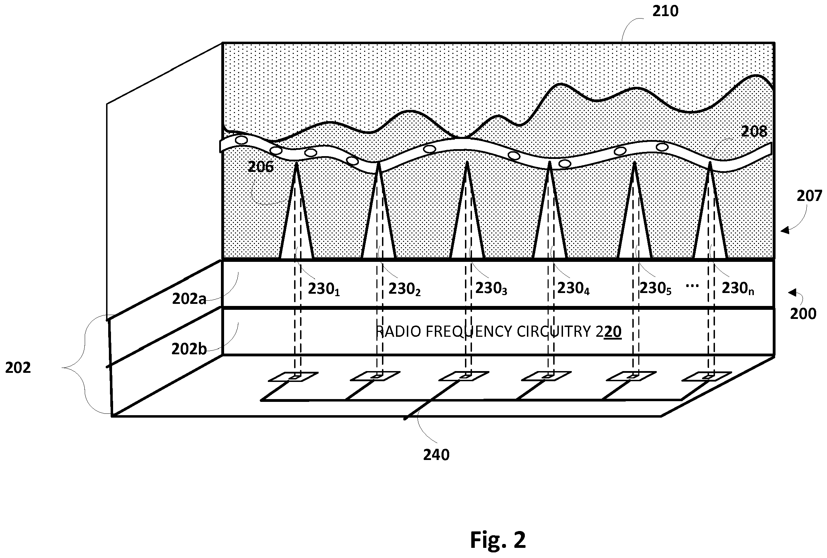

In other embodiments, such as that illustrated in Figure 2, the microneedles 206 may be oriented so that the tips of the microneedles 208 are pointing towards the surface 210 of the patient's skin. The embodiment of Figure 2 is otherwise very similar to that of Figure 1, and as such similar structure are number similarly with the first of the three digit number being replaced from a "1" in Figure 1 to a "2" in Figure 2. In some instances, the microneedles 206 may be oriented so that the tips of the microneedles are pointing towards the outer surface of the patient's skin and also perpendicular to the substrate 202. This arrangement may be present when, for example, the device is implanted into a patient's tissue 207. Colloquially, this may be referred to as the microneedles 206 being pointed "upward" but it is to be understood that the microneedle tips are pointed towards a patient's skin, and that may not be vertically "up," for example where the device is implanted in a patient's arm. Due to of the proximity of the microneedles/antennas to the surface of the skin the amount of bodily interference may be reduced, and therefore such an orientation may maximize the amount of electromagnetic radiation emitted from and received by the microneedles/antenna when in wireless communication with remote computing devices located external to the patient's body.

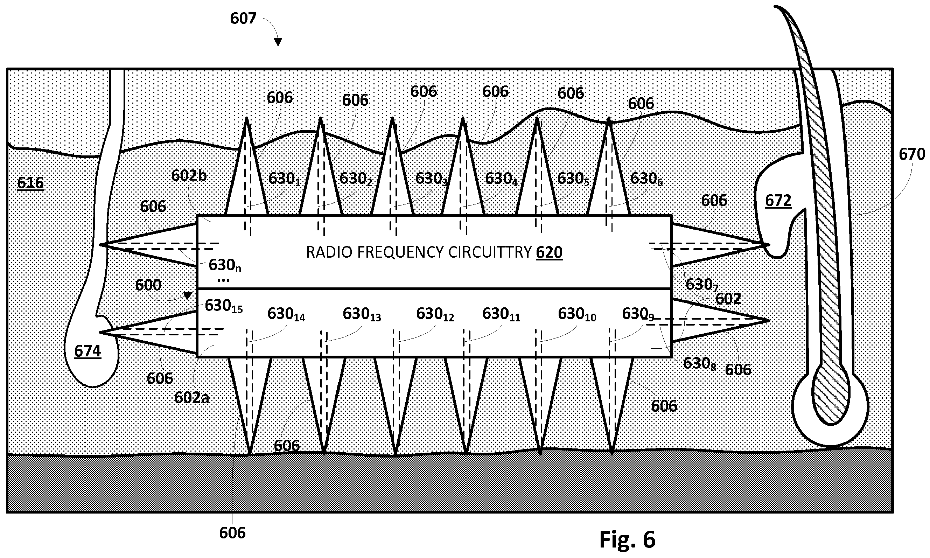

In some embodiments, such as illustrated in Figure 6, the microneedles may be oriented laterally with respect to the surface of the patient's skin, such that they are substantially parallel to the surface of the patient's skin. Such an orientation may be present when, for example, the device is inserted into a patient's tissue. Figure 6 depicts an alternative embodiment of an apparatus 600, similar to that depicted in Figs. 1, 2, and 4. However, in Figure 6 the apparatus 600 is an insertable device that has been inserted into tissue 607, *e.g*., within dermis 616 (although it may be inserted at various other depths within tissue 607). Additionally, unlike apparatus 100, 200, or 400, apparatus 600 includes a plurality of microneedles 606 that extend from the substrate layers 602a, 602b in multiple directions. In this example, microneedles 606 extend in directions both perpendicular and parallel to the skin surface. In such an orientation the long axis of the microneedle/antenna may be oriented to more effectively produce and pick up radio signals through the creation of a longer dipole. This may allow for efficient communication in even small devices, as in this configuration the microneedles themselves may effectively add to the physical dimension of the antenna. The antenna on the microneedle may, for example, be used to extend an antenna which is situated on the body of the device, by electrically connecting the antennas together.

Microneedles 606 may extend from the substrate layers 602a, 602b laterally, so as to be parallel to the skin surface. In Figure 6, for example, laterally extending microneedles 606 penetrate, on the right, a sebum gland 672 associated with a hair follicle 670. On the left, laterally extending microneedles 606 penetrate a sweat gland 674. Accordingly, in addition to drawing fluids from/administering fluids to capillaries, as was described above, in embodiments such as that depicted in Figure 6, fluids may be drawn from/injected into other anatomical components, such as sebum gland 672 and/or sweat gland 674. As described above, the microneedles 606 themselves may be fabricated on the silicon substrate, e.g., on layer 602a, 602_{b}, and each may define a micro-fluidic channel 630 that fluidly couples the tissue 607 of the patient with a corresponding conduit (e.g., a fluid interface, port, opening, etc.) (not illustrated in Figure 6) of the silicon substrate 602. The conduits may provide a fluid interface to, for instance, an additional fluid analysis layer (see Figure 4) and/or fluid pathways (e.g., tubing, hoses, etc.) to other destinations, such as a remote fluid analysis system. Each micro-fluidic channel 630 may allow for the collection of fluid from the tissue of a patient, for example blood; may allow for the insertion of fluid into the tissue, for example a pharmaceutical; or, may be used to both collect and insert varying fluids into the tissue of a patient. Furthermore, the microneedles 606 may be electrically coupled with the radio frequency circuitry 620 and radiate electromagnetic waves based on electrical currents generated by the radio frequency circuitry. In other words, one or more of the microneedles 606 may also function as an antenna. While the insertable embodiment depicted in Figure 6 includes microneedles 606 that extend both laterally and perpendicularly relative to the skin surface, that is not meant to be limiting. In various embodiments, insertable embodiments configured with selected aspects of the present disclosure may include only laterally-extending microneedles or only perpendicular-extending microneedles.

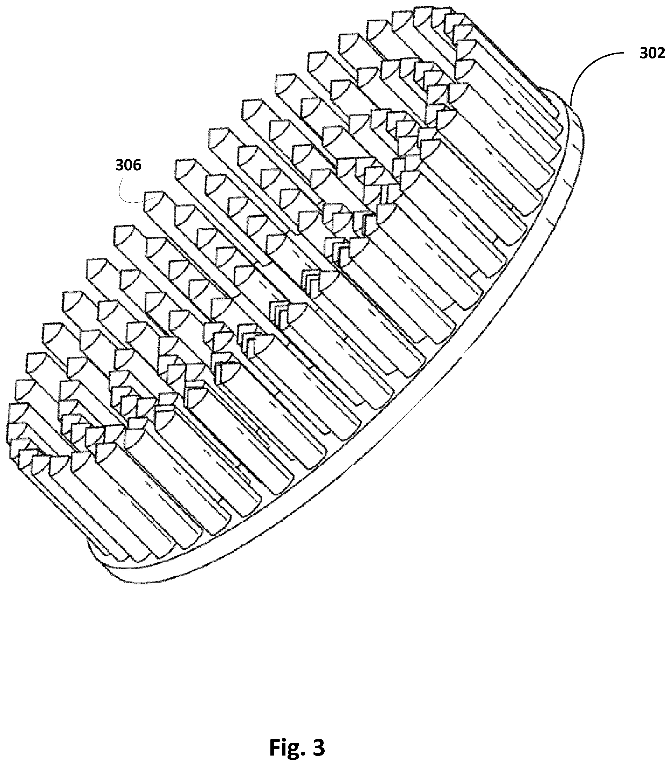

In still other embodiments, such as illustrated in Figure 3, the microneedles 306 may be arranged on the silicon substrate 302 in the form of concentric rings, e.g., following the profile of a corrugated horn antenna. Although not illustrated in Figure 3, other features disclosed herein may be incorporated into an embodiment of the apparatus taking the form illustrated in Figure 3 - a corrugated horn antenna. In general, horn antennas serve the same function for radio waves that acoustical horns do for sound - they provide a gradual transition, through their structure, to match the impedance of space so that radio waves transmit more efficiently. In particular, corrugated horn antennas are horns containing parallel slots or grooves, typically circular, covering the inside surface of a horn, transverse to the axis. In these embodiments, the continuous metal rings of a typically corrugated horn antenna may be replaced by rings of electrically interconnected microneedles as described herein. Such an arrangement of microneedles may provide a gain in radiation and improved directionality of the antenna and wireless communications. The size of a device incorporating the form of a corrugated horn antenna may vary based on the desired radio frequency to be used for communication. In some embodiments in which the desired radio frequency is 60 GHz, the corrugated horn antenna form may be about 10 millimeters in diameter with microneedles about 1250 microns long. In some embodiments, these dimensions may be larger than desirable. Operating at higher radio frequencies may be desired where it may be desired that the device be smaller than 10 millimeters in diameter. For example, using the 245 GHz radio frequency band may lead to fewer size constraints. A device operating at a radio frequency of 245 GHz may be about 2.5 millimeters in diameter with microneedles about 300 microns in length, although these sizes are not meant to be limiting.

In some embodiments, such as the embodiment illustrated in Figure 1, the microneedles are formed as a phased antenna structure 140. For example, in Figure 1, electrical contacts or electrodes 142₁₋ₙ may be electrically coupled with microneedles 106 (e.g., with conductive outer and/or inner surfaces of microneedles 106) on or within second layer 102b. Electrodes 142₁₋ₙ may be electrically coupled with each other via a circuit 144, which may comprise part of radio frequency circuitry 120. A signal from each of the plurality of microneedles 106 may be captured at a corresponding electrode 142 and combined with signals from the other microneedles 106 in order to achieve beamforming of at least some of the electromagnetic waves. Generally, beamforming allows for focusing the signal being emitted or in other words allows to steer the transmitted electromagnetic wavefront in a desired direction. Traditionally, a phase array antenna contains many radiating elements (here, the microneedles). Steered beams are formed when the phase of the signal emitted by each of the elements is shifted by a selected value so as to provide constructive interference steering the beams in the desired direction. Therefore, forming a phased antenna structure by combining the signals of the microneedles/antennas 106 allows for the signals for be focused in a particular direction. Furthermore, connecting multiple microneedles/antennas 106 to form a phased array antenna structure 140 may be hardware programmable, such that one can choose the best possible combination of the individual antennas to form the phased array structure for the desired purpose. In these embodiments the radio frequency circuitry 120 may comprise an application specific circuitry (ASIC) enabling an electronic steering of the transmitted and/or received electromagnetic waves, which can be realized by combining signals from one or more of the plurality of microneedle arrays to achieve beamforming of at least some of the electromagnetic waves.

Returning to the radio frequency circuitry 120 of Figure 1, the circuitry may be configured so as to facilitate one-way communication, such as where the microneedles/antennas 106 only wirelessly transmit information to a remote device. However, in other embodiments, the radio frequency circuitry may be configured to receive electrical current generated by the microneedles/antennas based on the incoming electromagnetic waves received from other remote devices and captured by the microneedles/antennas 106. These embodiments of the device may be capable of two-way wireless communication with remote devices, as the microneedles/antennas 106 may be capable of both transmitting and receiving wireless signals. In other embodiments, the microneedles/antennas 106 may be capable only of receiving wireless signals, e.g., to cause administration of a drug through microneedles 106.

It may be desirable to increase the sensitivity of the antennas so that they may transmit and/or receive signals from longer distances and/or signals with great clarity. In such instances, the surface area of metal (or more generally, conductive material) may be increased. For example, each microneedle 106 may be coated partially or completely with metal. Types of metal that used may include, but are not limited to, silver, gold, copper, aluminum, zinc, nickel, iron, tin, and/or any metal, or combination thereof, known in the art to be conductive. The metal coating may be applied during a final metallizing step, which may also improve the manufacturability of the device. In some embodiments, the metal coating may be placed on the exterior surface of each of the microneedles/antennas. In other embodiments, the metal coating may be place on an interior surface, e.g., that defines the micro-fluidic channel of each of the microneedles. With a metal coating, an individual microneedle/antenna may have a length of about 200 to about 500 microns. However, there may be instances in which it is desired that the dimensions of a 60GHz antenna be about 2500 microns. Therefore, in some embodiments, several microneedles/antennas may be connected together to form a desired dimension for use in a 60GHz band.

In some embodiments, the wearable or insertable device further contains one or more through silicon vias. Through silicon vias are electrical connections that may pass through silicon, and may be used as an alternative to bonding wires. Through silicon vias may have a higher density, as compared to other techniques, and thus may not require as long of a connection. In various embodiments, the one or more through silicon vias of the wearable or insertable device may fluidly couple the micro-fluidic channels (130 in Figure 1) of the microneedles/antennas through the layer of silicon substrate that supports the microneedles (e.g., 102a in Figure 1) to the conduit (132 in Figure 1) of the silicon substrate. In some embodiments, the length of the microneedles/antenna may be extended, e.g., to increase transmission and/or reception of signals from longer distances and/or provide increase signal clarity. This extension may be achieved in some embodiments by elongating a microneedle/antenna element through the silicon substrate 102 (e.g., through layer 102b) using one or more metallic through silicon vias. In some such embodiments, the total length of the antennas may reach about 250 to about 1500 microns.

In some embodiments, such as illustrated in Figure 4, the micro-fluidic channels 430₁₋ₙ of the silicon substrate 402 leads to an additional substrate layer 402c. As with the previously described embodiments, the substrate may include multiple layers, for example a layer that supports a plurality of microneedles 402ₐ, a layer that the radio frequency circuitry 420 is incorporated into 402_{b}, and in some embodiments an additional layer 402_{c}. This additional layer may be a fluid analysis layer 430, as illustrated in Figure 4. This fluid analysis layer may analyze fluid extracted from the patient's tissue for presence/absence or levels of various biomarkers. As a non-limiting example, the fluid analysis layer may be used to analyze the level of cholesterol or glucose in a patient's blood. The additional layer may include reservoir or fluid containing chamber (not depicted in Figure 4). Such a reservoir or fluid containing chamber may be used for storing fluid for injection into the tissue of a patient. As non-limiting example, this reservoir or fluid containing chamber may contain insulin, or another pharmaceutical agent to be injected into the patient.

In some instances the presence of fluids in the microneedles-whether the fluids are bodily fluids extracted from the tissue of a patient or pharmaceuticals for injection into the patient-may negatively impact wireless communication with the antennas. Therefore, in some embodiments, the wearable/insertable device may further contain a fluid detection sensor, which is capable of determining if fluid is present or absent in the micro-fluidic channels of the microneedles. Where fluid is present the fluid detection sensor may provide a signal indicating as much. Such a signal may prevent the transmission or reception of data. Alternatively, where no fluid is contained in the microneedles, the fluid detection signal may provide a signal indicating the absence of fluid. Where no fluid is present a signal by the fluid detection sensor may indicate to the radio frequency circuitry that the antenna structure(s) may be used for transmitting and receiving radio frequency signals. In still other embodiments, it may be desirable to maintain constant fluid flow through the microneedles, such as where a pharmaceutical needs to be constantly delivered to the tissue of a patient. In such instances, a fluid detection sensor may not be desirable.

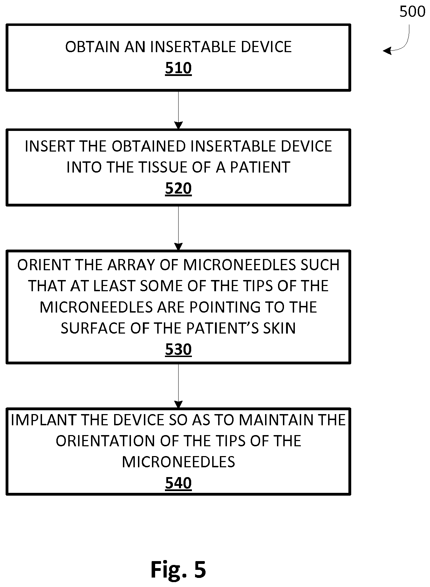

Referring now to Figure 5, an exemplary method 500 for implanting a device into the tissue of a patient that may be practiced, for instance, using the apparatuses (100, 200, 300, 400) described herein. While operations of method 500 are depicted in a particular order, this is not meant to be limiting. In various embodiments, one or more operations may be added, omitted, and/or reordered.

At block 510, an insertable device such as depicted in Figure 2, Figure 3, Figure 4, Figure 6, or otherwise described herein is obtained. The particular embodiment(s) of the insertable device may be dependent on the desired use of the device and the needs of a particular patient. For example, some patients may only require the device be capable of one-way wireless communication with a remote device, while other patients may require the device be capable of two-way wireless communication.

The method may further comprises a step illustrated by block 520, wherein the obtained device is inserted into the tissue of the patient. The device may be inserted at varying depths of tissue depending on the desired use of the device. Furthermore, the device may be inserted at varying locations of tissue on/in a patient's body depending on the desired use of the device. For example, the device may be inserted into the dermal layers of a patient's arm, leg, chest area, neck, or anywhere else suitable for the desired use.

The method may further include a step of orienting (530) the array of microneedles illustrated at block 530, wherein the inserted device is oriented so that at least some of the tips of the microneedles/antennas are pointing to the surface of the patient's skin. Alternatively, in some embodiments, a device may be oriented so that the microneedles are positioned essentially parallel to a patient's skin surface, as illustrated in Figure 6. Because of the proximity of the microneedles/antennas to the surface of the skin the amount of bodily interference may be reduced. Alternatively, an orientation where the microneedles are parallel to the surface of the skin (e.g., as shown in Figure 6) may maximize the effective size of the antenna, and such orientations may maximize the amount of radiation emitted from and received by the microneedles/antenna when in wireless communication with remote computing devices located external to the patient's body.

Further at block 540, the oriented device may be implanted into the patient's tissue, so as to maintain the desired orientation in which the device was placed at block 530. Implanting the device may maintain the quality of the transmission and reception of wireless data. Further, implanting the device into the tissue of the patient may prevent, or minimize, the dislocation of the device from the desired placement/orientation.

The method 500 may further comprises a step of fluid detection, wherein by means of a fluid detection sensor the presence of fluid in one or more of the plurality of microneedles is detected. The sensor is further arranged to active the radio frequency circuitry when fluid is present. Further, this activation step may comprise generating, by the radio frequency circuitry, the signal in response to determining, based on the signal from the fluid detection sensor, that there is no fluid in the microfluidic channels of one or more microneedles of the array of the microneedles.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03. It should be understood that certain expressions and reference signs used in the claims pursuant to Rule 6.2(b) of the Patent Cooperation Treaty ("PCT") do not limit the scope.

## Claims

1. A wearable or insertable device (100, 200, 400, 600), comprising:
a substrate (102, 202, 302, 402, 602) that is affixable to or insertable into tissue (107, 607) of a patient;
radio frequency circuitry (120, 220, 420, 620) coupled to the substrate, wherein the radio frequency circuitry is operable to generate or process a signal with a frequency that is modulated to carry information; and
an array of microneedles (106, 206, 306, 406, 606) extending from at least one surface of the substrate, wherein one or more microneedles of the array of microneedles defines a micro-fluidic channel (130, 230, 430, 630) that is adapted to fluidly couple the tissue of the patient with a conduit (132) of the substrate, and wherein one or more microneedles of the array of microneedles are electrically coupled with the radio frequency circuitry and are adapted to radiate or process electromagnetic waves based on the signal.

2. The wearable or insertable device of claim 1, wherein the substrate comprises a multi-layer silicon substrate that includes:
a first layer (102b, 202b, 402b,602b) comprising the radio frequency circuitry (120, 220, 420, 60); and
a second layer (102a, 202a, 402a, 602a) acting to support the array of microneedles.

3. The wearable or insertable device of claim 2, where in the substrate further comprises one or more through silicon vias, which are arranged to fluidly couple the micro-fluidic channels (130, 230, 430, 630) of the array of microneedles (106, 206, 306, 406, 606) by extending through the first layer to the conduit (132).

4. The wearable or insertable device of claim 3, wherein the substrate (402) further comprises an additional layer of a fluid analysis layer (402c), which is coupled to the conduits (132) and is arranged to analyze fluid extracted through the micro-fluidic channels (130, 230, 430, 630) of the array of microneedles (106, 206, 306, 406, 606).

5. The device of claim 3 further comprising, a fluid chamber coupled to the conduits (132) and arranged to contain fluid for injection into tissue (107, 607) via the micro-fluidic channels (130, 230, 430, 630) of the array of microneedles (106, 206, 306, 406, 606).

6. The wearable or insertable device of claim 1, wherein the array of microneedles (106, 206, 306, 406, 606) are formed as a phased antenna structure (140) arranged to combine signals from one or more of the plurality of microneedle arrays to achieve beamforming of at least some of the electromagnetic waves.

7. The device of claim 1, wherein the plurality of microneedles (106, 206, 306, 406, 606) are oriented either perpendicularly or parallel to the substrate (102, 202, 302 402, 602).

8. The device of claim 1, wherein the plurality of microneedles (106, 206, 306, 406, 606) are laterally oriented with respect to a patient's skin surface.

9. The device of claim 1, wherein each of the plurality of microneedles (106, 206, 306, 406, 606) further comprise a metal coating applied to either an interior surface an exterior surface of the micro-fluidic channel (130, 230, 430, 630) of each of the plurality of microneedles (106, 206, 306, 406, 606).

10. The device of claim 1, wherein at least some of the plurality of microneedles, wherein each microneedle is arranged to act as antenna element and extends through the substrate (102, 202, 302 402, 602) by way of metallic vias.

11. The device of claim 10, wherein the antenna element is extended to a length of about 250 to about 1500 microns.

12. The device of claim 1, wherein the device further comprises a fluid detection sensor arranged to detect presence of fluid in the plurality of microneedles (106, 206, 306, 406, 606) and to activate the radio frequency circuitry (120, 220, 420, 620) when fluid is present.

13. The device of claim 12, wherein the radio frequency circuitry (120, 220, 420, 620) is further arranged to process the signal in response to a determination, based on a signal from the fluid detection sensor, that there is no fluid in the micro-fluidic channels of the array of microneedles (106, 206, 306, 406, 606).

14. The device of claim 1, wherein the array of microneedles (106, 206, 306, 406, 606) are arranged in concentric rings following a profile of a corrugated horn antenna.

15. A method comprising:
obtaining (510) an insertable device (100, 200, 400, 600), wherein the device comprises:
a substrate (102, 202, 302, 402, 602) that is affixable to or insertable into tissue (107, 607) of a patient;
radio frequency circuitry (120, 220, 420, 620) disposed on the substrate, wherein the radio frequency circuitry is operable to generate or receive a signal a frequency that is modulated to carry information; and
an array of microneedles (106, 206, 306, 406, 606) extending from at least one surface of the substrate, wherein one or more microneedles of the array of microneedle defines a micro-fluidic channel (130, 230, 430, 630) that fluidly couples the tissue of the patient with a conduit (132) of the substrate, and wherein one or more microneedles of the array of microneedles are electrically coupled with the radio frequency circuitry and radiate electromagnetic waves based on the signal.
